# EUROPÄISCHE PATENTANMELDUNG

(11) **EP 2 548 526 A1**
(43) Veröffentlichungstag der Anmeldung: **23.01.2013**
(21) Anmeldenummer: 12004760.0
(22) Anmeldetag: 26.06.2012
(51) Int. Cl.: A61B 17/88, A61B 17/70, A61F 2/46

(54) **Applikator-Kit**

(30) Priorität: 20.07.2011 DE 102011108010
(71) Anmelder: Heraeus Medical GmbH, 61273 Wehrheim (DE)
(72) Erfinder: Villone, Dr. Daniela, 61350 Bad Homburg (DE); Vogt, Dr. Sebastian, 99092 Erfurt (DE)
(74) Vertreter: Panten, Kirsten

(57) **Zusammenfassung**

Die Anmeldung betrifft einen Applikator zur Applikation von partikulären Knochenersatzmaterialien, autologer oder heterologer Spongiosa, demineralisierter Knochenmatrix oder deren Gemischen sowie ein Applikator-Kit, das einen solchen Applikator enthält. Der Applikator (1) enthält ein hohles Rohr (12), das an einem Ende einen Lamellenverschluss (13) aufweist, so dass das Knochenersatzmaterial sicher von dem Lamellenverschluss zurückgehalten wird und vor der Applikation nicht unbeabsichtigt das Operationsfeld kontaminieren kann. Am anderen Ende weist der Applikator ein erstes Befestigungsmittel (15) auf, das mit einem zweiten Bauteil in Eingriff gebracht werden kann. Das zweite Bauteil enthält einen Stab (2) mit einem Handgriff (21) an einem Ende und einem Kolben (22) am gegenüberliegenden Ende. Der Stab ist in einer entlang des Stabs (2) beweglichen Führungshülse angeordnet, die ein zweites Befestigungsmittel (23) aufweist, das mit dem Befestigungsmittel (15) des Applikators (1) reversibel verbunden werden kann. Der Applikator ist Teil eines Applikator-Kits, das außerdem einen Mischbecher enthält, der anstelle des Stabs mit dem Rohr (12) verbunden werden kann, so dass das zu applizierende Material vor der Applikation einfach in das Rohr (12) eingefüllt werden kann.

## Beschreibung

Die Erfindung betrifft einen Applikator zur Applikation von partikulären Knochenersatzmaterialien, autologer oder heterologer Spongiosa, demineralisierter Knochenmatrix oder deren Gemischen sowie ein Applikator-Kit, das einen solchen Applikator enthält.

Knochendefekte werden nach wie vor mit autologer Spongiosa aufgefüllt. Autologe Spongiosa kann jedoch nur in relativ geringen Volumina vom Patienten gewonnen werden. Dadurch sind der Verwendung von autologer Spongiosa trotz des sehr guten Einwachsverhaltens Grenzen gesetzt. Eine Möglichkeit zur Lösung dieses Problems besteht darin, die autologe Spongiosa mit synthetischen oder semisynthetischen Knochenersatzmaterial zu augmentieren. Bei den Knochenersatzmaterialien werden gegenwärtig am häufigsten Calciumphosphate wie ß-Tricalciumphosphat, Hydroxylapatit und deren Gemische eingesetzt. Diese liegen im Allgemeinen als poröse oder auch als nichtporöse Granula vor. Daneben sind auch auf Calciumsulfat basierende partikuläre Knochenersatzmateriallen bekannt.

Die Vermischung von autologer Spongiosa mit partikulären Knochenersatzmaterialien wird üblicherweise im Operationssaal durch einfaches Vermischen der Spongiosa mit dem Knochenersatzmalerial in einem sterilen Gefäß durchgeführt.

Es wurde weiterhin eine Reihe von Vorrichtungen zum Vermischen von Knochenersatzmaterialien mit Spongiosa oder Blut oder plättchenreichen Plasma (PRP) beschrieben.

Im WO 2002/068010 A1 wurde eine Vorrichtung vorgeschlagen, bei der ein Knochenmarkaspirat mit einem porösen Knochenersatzmaterial einfach vermischt werden kann. Das aspirierte Knochenmark wird mit zwei Spritzen durch das Knochenersatzmaterial durchgedrückt oder durchgesaugt.

Eine ähnlich wirkende Vorrichtung ist aus US 6,049,026 A1 bekannt. Diese Vorrichtung besteht aus einem Behälter zur Aufnahme des Knochenersatzmaterials und einem oberhalb des Behälters angeordneten zweiten Behälter. Der zweite Behälter dient zur Aufnahme des aspirierten Knochenmarks. Weiterhin ist ein dritter Behälter unterhalb des ersten Behälters angeordnet. Zwischen dem ersten und dem zweiten Behälter ist ein zweites Ventil angeordnet. Ein weiteres Ventil befindet sich zwischen dem ersten und dem dritten Behälter. Das aspirierte Knochenmark fließt nach Öffnung des ersten Ventils in den ersten Behälter und tränkt das Knochenersatzmaterial. Danach wird das zweite Ventil geöffnet und das überschüssige Knochenmark fließt in den dritten Behälter.

Eine sehr einfache Vorrichtung wurde in US 7,776,594 B2 offen gelegt. Der Mischbehälter besteht aus einem Rohr mit zwei Schraubkappen an den Enden. Das Knochenersatzmaterial wird einfach in das Rohr eingebracht und anschließend Knochenmarkaspirat zugespritzt.

US 6,793,660 B2 beschreibt eine Vorrichtung zum Einbringen von Knochenersatzmaterialien, die aus einer Spritze mit Innengewinde und einem drehbaren Stößel mit Außengewinde besteht. Das Knochenersatzmaterial wird einfach durch Drehen des Stößels in Richtung des Spritzenkopfs herausgepresst. Problematisch ist die sehr große Auspresskraft dieses Systems, weil dadurch granuläres Knochenersatzmaterial mechanisch geschädigt werden kann. Weiterhin ist es möglich, dass bei Verwendung von autologer Spongiosa oder Gemischen aus autologer Spongiosa mit Knochenersatzmaterial durch die große Krafteinwirkung die Zellen der Spongiosa geschädigt werden können.

Im Operationssaal tritt immer wieder das Problem auf, dass das Einbringen des partikulären Knochenersatzmaterials oder auch dessen Gemische mit Spongiosa oder Blut oder plättchenreichem Plasma nur mit Löffeln, Spateln oder modifizierten Spritzen, denen der Spritzenkopf abgeschnitten wurde, nur schwer möglich ist. Insbesondere das Auffen von Kavitäten in langen Röhrenknochen ist problematisch. Daher wäre ein langer spritzenartiger Applikator sinnvoll. Weiterhin es hygienisch problematisch, Gemische mit Blut oder Blutbestandteilen in modifizierte Spritzen mit Hilfe von Löffeln und Spritzen zu füllen, ohne dass eine Kontamination des OP-Personals und des Operationsfeldes auftritt. Es wurde bisher kein einfacher, sicherer und hygienischer Transfer von Knochenersatzmaterilaien oder deren Gemischen aus Mischbehältem in geeignete spritzenartige Applikatoren bekannt.

Der Erfindung liegt die Aufgabe zu Grunde, ein möglichst einfaches Applikator-Kit zur Applikation von partikulären Knochenersatzmaterialien, autologer oder heterologer Spongiosa, demineralisierter Knochenmatrix oder deren Gemischen zu entwickeln, welches die Nachteile des Standes der Technik überwindet.

Die Aufgabe der Erfindung wurde durch einen Applikator nach Anspruch 1 und ein Applikator-Kit nach Anspruch 10 gelöst.

Die Erfindung betrifft einen Applikator, aufweisend ein hohles Rohr, das an einem Ende einen Lamellenverschluss aufweist, und am anderen Ende ein erstes Befestigungsmittel aufweist, einen Stab, der an einem Ende einen Handgriff und am gegenüberliegenden Ende einen Kolben aufweist und in einer entlang des Stabs beweglichen Führungshülse angeordnet ist, wobei die Führungshülse einen Durchmesser hat, der kleiner oder gleich dem Innendurchmesser des hohlen Rohrs ist, und ein zweites Befestigungsmittel aufweist, das mit dem Befestigungsmittel des Applikators reversibel verbunden werden kann.

Sie betrifft außerdem ein Applikator-Kit, enthaltend einen Applikator nach einem der vorangehenden Ansprüche und einen Mischbecher, der an seiner Unterseite zumindest eine Öffnung besitzt, die auf der Außenseite von einer zweiten Hülse umgeben ist, wobei die zweite Hülse einen Durchmesser hat, der kleiner oder gleich dem Innendurchmesser am hinteren Ende des Rohrs ist, und ein drittes Befestigungsmittel aufweist, das mit dem Befestigungsmittel des Applikators reversibel verbunden werden kann.

Erfindungsgemäß bedeutet "hinteres Ende" des Applikators das Ende, an dem der Stab eingeführt wird, während "vorderes Ende" das Ende mit dem Lamellenverschluss bedeutet, wo das granuläre Knochenersatzmaterial herausgedrückt wird.

Grundsätzlich sind für das Rohr des Applikators unterschiedliche Querschnitte denkbar, bevorzugt ist jedoch ein Rohr mit einem runden Querschnitt. Gleichermaßen kann auch der aufgeweitete Bereich unterschiedliche Querschnitte haben. Der Querschnitt des aufgeweiteten Bereichs muss nicht die gleiche Form wie der Querschnitt des Rohrs haben, jedoch ist die Querschnittsfläche größer als die des Rohrs. Von Vorteil ist ebenfalls ein runder Querschnitt.

In dem Applikator sind Stab und Rohr ähnlich wie in einer medizinischen Spritze angeordnet. Jedoch werden beide Bauelemente mit Befestigungsmitteln aneinander fixiert. Dabei können die unterschiedlichsten Befestigungsmittel zum Einsatz kommen. Als besonders vorteilhaft hat sich ein Bajonettverschluss erwiesen, da er schnell geschlossen und ebenso schnell wieder gelöst werden kann. Es sind jedoch auch andere Befestigungsmittel denkbar, wie zum Beispiel ein Schraubverschluss.

Die Befestigungsmittel sind am hinteren Rohrende und an einer Führungshülse des Stabs angeordnet. Innerhalb der Führungshülse kann der Stab vor und zurück bewegt werden. Bevorzugt ist das hintere Ende des Rohrs aufgeweitet und bildet einen Zylinderabschnitt mit größerem Durchmesser als das Rohr. Die Führungshülse hat in diesem Fall bevorzugt einen Außendurchmesser, der dem Innendurchmesser der Aufweitung entspricht oder etwas kleiner ist. Die Befestigungsmittel sind dann am Außenumfang der Führungshülse und am Innenumfang des Zylinderabschnitts angebracht.

Der Lamellenverschluss ist ideal, um eine Masse, wie autologe Spongiosa auszugeben. Erst wenn Druck auf die Masse ausgeübt wird, drückt sie die Lamellen nach außen und gibt das Knochenersatzmaterial aus. Bevorzugt bilden die Lamellen eine Spitze. Der Abstand zwischen den Lamellen sollte an das Granulat angepasst sein und sollte im geschlossenen Zustand so klein sein, dass das Material nicht austritt. Durch Aufbiegen der Lamellen entsteht eine Öffnung, die so groß ist, dass das Material austreten kann. Es versteht sich somit auch, dass "geschlossener Zustand" nicht bedeuten muss, dass die Lamellen einen gegenüber Flüssigkeiten der gas dichten Verschluss bilden, es bedeutet vielmehr, dass das Knochenersatzmaterial nicht austreten kann.

Bevorzugt hat das hohle Rohr des Applikators einen Außendurchmesser von 4 bis 15 mm, besonders bevorzugt von 8 bis 13 mm und ganz besonders bevorzugt von 11 bis 13 mm. Die Länge vom vorderen Ende einschließlich Lamellenverschluss bis zum Ende der zylinderförmigen Aufweitung beträgt bevorzugt von 20 bis 40 cm, besonders bevorzugt 25 bis 35 cm, und ganz besonders bevorzugt von 28 bis 32 cm.

Damit ist der Applikator hinsichtlich seiner Länge und dem Durchmesser so beschaffen, dass er in Röhrenknochen, wie Tibia und Femur, problemlos eingeführt werden kann und dass partikuläres Material dann problemlos auch in die am weitesten vom Zugang entfernten Kavitäten von Röhrenknochen sicher eingebracht werden kann und damit eine weitgehende Auffüllung möglich ist.

Vorteilhaft ist der Applikator aus einem Material mit einer gewissen Elastizität gefertigt. Die Materialien sollten biokompatibel sein, wie zum Beispiel Polypropylen. Durch Wahl eines elastischen Materials kann der Applikator beim Einführen den anatomisch vorgegebenen Krümmungen der Röhrenknochen folgen.

Bevorzugt ist in dem Mischgefäß ein Mischlöffel oder Mischspatel angeordnet, wobei ein Mischspatel besonders bevorzugt ist, der am Ende des Spatels einen flächigen Stopfer besitzt, der senkrecht zur Achse des Mischspatels steht und dessen Querschnittfläche kleiner oder gleich der Querschnittsfläche der Öffnung des Mischbechers ist.

Die Applikation von partikulären Knochenersatzmaterialien, autologer oder heterologer Spongiosa, demineralisierter Knochenmatrix oder deren Gemischen mit dem Applikator-Kit erfolgt, indem zuerst der Mischbecher mit dem Knochenersatzmaterialien, autologer oder heterologer Spongiosa, demineralisierter Knochenmatrix oder deren Gemischen befüllt wird. Dabei verschließt der Schieber die Öffnung des Mischbechers. Der Mischbecher wird mit dem Stutzen in die zylinderförmige Aufweitung eingebracht und die Befestigungsmittel werden reversibel miteinander verbunden. Anschließend wird der Schieber gezogen, so dass die Öffnung des Mischbechers frei wird und das Material in das hohle Rohr des Applikators fällt. Gegebenenfalls kann das Material auch mit dem Stopfer des Mischspatels in den Hohlraum des Applikators gestopft werden. Anschließend wird durch Lösen der Befestigungsmittel die Verbindung zwischen dem Applikator und dem Mischbecher gelöst. Anschließend wird der Stab mit Führungshülse eingesetzt, so dass sich die Führungshülse in der Aufweitung des Applikators befindet. Die Befestigungsmittel an Rohr und Stab werden miteinander in Eingriff gebracht. Durch Schieben des Stabs wird das Material in Richtung Lamellenverschluss geschoben, der Lamellenverschluss biegt sich auf und das Material wird ausgegeben.

Das erfindungsgemäße Applikator-Kit weist gegenüber dem Stand der Technik eine Menge Vorteile auf.

Vor der eigentlichen Applikation ermöglicht das Applikator-Kit, partikuläre Knochenersatzmaterialien mit autologer oder heterologer Spongiosa oder demineralisierter Knochenmatrix zu vermischen und sauber und sicher in den Applikator zu transferieren.

Der Applikator kann einfach und sicher per Hand betätigt werden kann. Durch einfaches Drücken gegen den Stab kann das Knochenersatzmaterial aus dem Applikator herausgedrückt werden, ohne dass das partikuläre Knochenersatzmaterial durch Scherung an scharfen Kanten beschädigt wird.

Auf diese Weise werden einige Probleme unterbunden, die bei der Verwendung von herkömmlichen Applikatoren auftreten. Das Knochenmaterial wird sicher von dem Applikationsrohr aufgenommen und von dem Lamellenverschluss zurückgehalten, so dass das Knochenersatzmaterial vor der Applikation nicht unbeabsichtigt aus dem Applikator heraustreten kann und das Operationsfeld kontaminiert. Ein Entfernen von Granula aus dem Knochen umgebende Weichgewebe wäre störend und würde die Operationszeit unnötig verlängern. Durch Fixieren des Stabs im Rohr wird außerdem verhindert, dass dieser in das Operationsfeld oder auf den Boden des Operationssaals fällt, und somit der Operationsverlauf gestört wird.

Im Folgenden wird die Erfindung anhand der beiliegenden Zeichnungen beispielhaft beschrieben. Es zeigen:
Fig. 1 eine bevorzugte Ausführungsform des Applikators im Ausschnitt und Teilschnitt,
Fig. 2 einen weiteren Ausschnitt des Applikators, und
Fig. 3 einen Mischbecher eines Applikator-Kits in perspektivischer Darstellung.

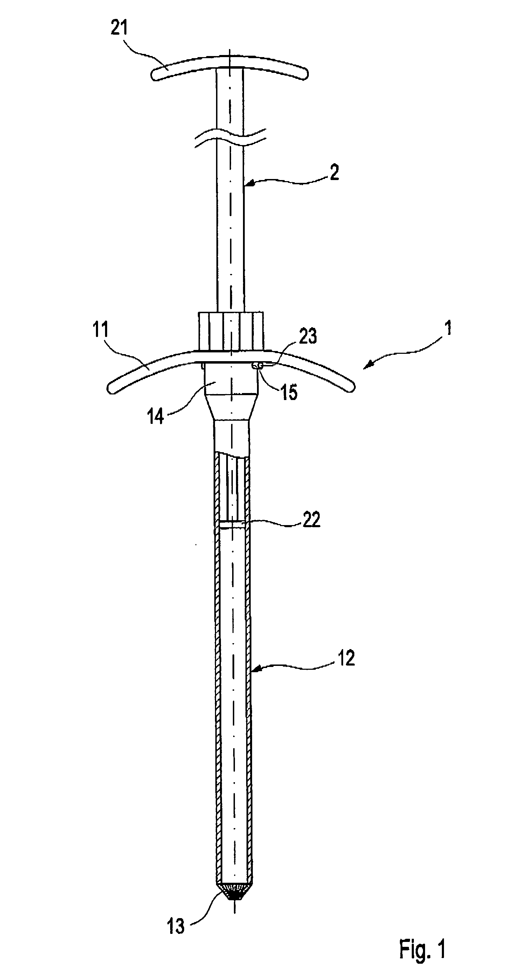

Fig. 1 zeigt den Applikator 1 im Teilschnitt. Er weist ein hohles Rohr 12 auf, in dem sich die autologe Spongiosa befindet. Am hinteren Ende hat das Rohr 12 einen aufgeweiteten zylindrischen Bereich 14, in den eine Führungshülse eingesetzt ist. Die Führungshülse dient zur Führung eines Stabes 2, an dem ein Kolben 22 befestigt ist. Die Führungshülse weist einen Stift 23 auf, der mit einer L-förmigen Ausnehmung 15 in dem aufgeweiteten zylindrischen Bereich 14 zusammenwirkt..

Indem Druck auf den Stab 2 ausgeübt wird, wird der an dem Stab angeordnete Kolben 22 nach vom verschoben und die autologe Spongiosa gegen einen Lamellenverschluss 13 gedrückt. Der Lamellenverschluss 13 ist aus einem elastischen Material gefertigt, so dass die einzelnen Lamellen nach außen gebogen werden, wenn die Spongiosa gegen die Lamellen gedrückt wird.

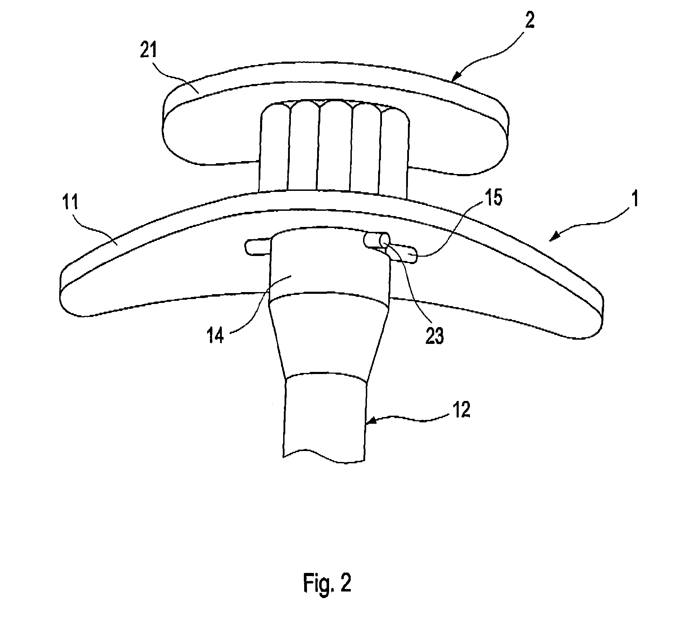

Fig. 2 zeigt einen Abschnitt des Applikators 1, in dem Griff und Befestigungsmittel hervorgehoben werden. Der Stab 2 ist vollständig in den Applikator 1 eingeschoben, so dass nur noch der Griff 21 zu sehen ist. Die Stifte 23, die an der Führungshülse befestigt sind, sind in der L-förmigen Ausnehmung 15 fixiert. Um die Handhabung zu vereinfachen, ist an der Aufweitung 14 ein zweiter Griff 11 angebracht. Der Anwender kann auf diese Weise Kraft auf den Stab 2 ausüben, indem er die beiden Griffe zusammen zieht.

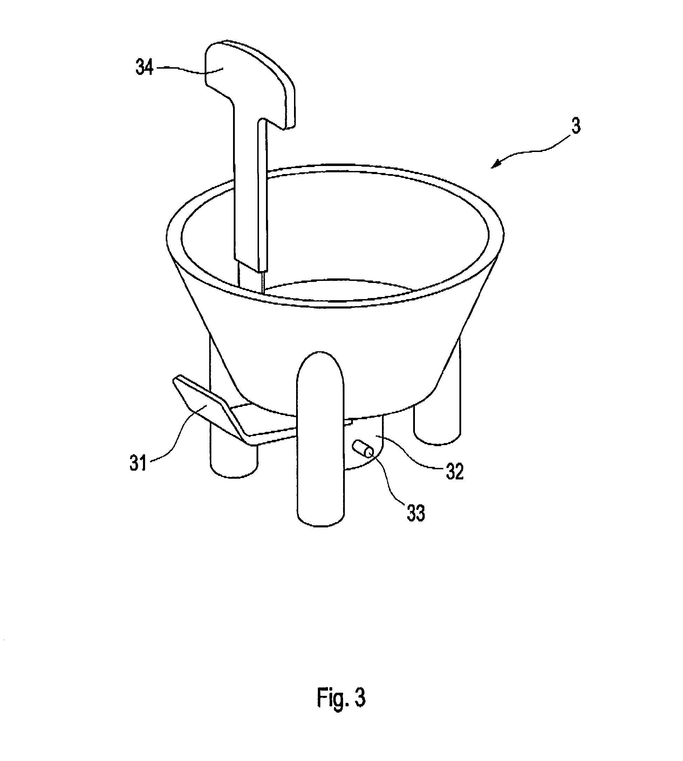

Bevor das Applikatorrohr 12 mit dem Stab 2 zusammengesetzt wird, muss es gefüllt werden. Dazu wird es mit einem Mischbehälter oder Mischbecher 3 verbunden, wie er in Fig. 3 gezeigt ist. Der Mischbecher 3 weist ein mit dem Stab 2 identisches Befestigungsmittel 33 auf, in diesem Fall ein Stift für einen Bajonettverschluss, über das der Mischbecher 3 fest mit dem Applikatorrohr 12 verbunden werden kann. Der Mischbecher 3 ähnelt einem Trichter, wobei die Ablauföffnung durch einen Schieber 31 verschließbar ist. Nachdem das einzufüllende Material in dem Mischbecher 3 mit Hilfe eines Spatels 34 gemischt worden ist, wird der Schieber 31 geöffnet und das Material mit Hilfe des Spatels 34 durch die Ablauföffnung und Ablaufrohr 32 in das Rohr gedrückt.

### Bezugszeichenliste

- 1: Applikator
- 11: Handgriff
- 12: Rohr
- 13: Lamellenverschluss
- 14: aufgeweiteter Bereich
- 15: erstes Befestigungsmittel, Führungsnut

- 2: Stab
- 21: Handgriff
- 22: Kolben
- 23: zweites Befestigungsmittel, Stift

- 3: Mischbecher
- 31: Verschluss, Schieber
- 32: Ausgaberohr
- 33: drittes Befestigungsmittel, Stift
- 34: Mischspatel

## Patentansprüche

1. Applikator (1) für Knochenersatzmaterial, aufweisend
ein hohles Rohr (12), das an einem vorderen Ende einen Lamellenverschluss (13) aufweist, und an einem hinteren Ende mit einem ersten Befestigungsmittel (15) ausgestattet ist, und einen Stab (2), der an einem Ende einen Handgriff (21) und am gegenüberliegenden Ende einen Kolben (22) aufweist und in einer entlang des Stabs (2) beweglichen Führungshülse angeordnet ist, wobei die Führungshülse einen Durchmesser hat, der kleiner oder gleich dem Innendurchmesser des Rohrs (12) ist, und ein zweites Befestigungsmittel (23) aufweist, das mit dem Befestigungsmittel (15) des Applikators (1) reversibel verbunden werden kann.

2. Applikator nach Anspruch 1 **gekennzeichnet durch**
einen aufgeweiteten Bereich (14) am hinteren Ende des Rohrs, wobei die Führungshülse einen Durchmesser hat, der kleiner oder gleich dem Innendurchmesser der aufgeweiteten Bereichs (14) ist.

3. Applikator nach Anspruch 1 oder 2, **dadurch gekennzeichnet, dass**
das erste Befestigungsmittel (15) und das zweite Befestigungsmittel (23) zusammen einen Bajonettverschluss bilden.

4. Applikator nach Anspruch 1 oder 2, **dadurch gekennzeichnet, dass**
das erste Befestigungsmittel (15) und das zweite Befestigungsmittel (24) zusammen einen Schraubverschluss bilden.

5. Applikator nach einem der vorangehenden Ansprüche, **dadurch gekennzeichnet, dass** der Lamellenverschluss (13) eine Spitze bildet.

6. Applikator nach einem der vorangehenden Ansprüche, **dadurch gekennzeichnet, dass**
das hohle Rohr (12) einen Außendurchmesser von 4 bis 15 mm.

7. Applikator nach Anspruch 6, **dadurch gekennzeichnet, dass**
das hohle Rohr (12) einen Außendurchmesser von 8 bis 13 mm.

8. Applikator nach einem der vorangehenden Ansprüche, **dadurch gekennzeichnet, dass**
das hohle Rohr (12) vom vorderen Ende einschließlich Lamellenverschluss (13) bis zum hinteren Ende eine Länge von 20 bis 40 cm hat.

9. Applikator nach einem der vorangehenden Ansprüche, **dadurch gekennzeichnet, dass**
das hohle Rohr vom vorderen Ende einschließlich Lamellenverschluss (13) bis zum hinteren Ende eine Länge von 25 bis 35 cm hat.

10. Applikator-Kit, **gekennzeichnet durch**
einen Applikator (1) nach einem der vorangehenden Ansprüche und
einen Mischbecher (3), der an seiner Unterseite zumindest eine Öffnung besitzt, die auf der Außenseite von einem Ausgaberohr (32) umgeben ist, wobei das Ausgaberohr (32) einen Durchmesser hat, der kleiner oder gleich dem Innendurchmesser des aufgeweiteten Bereichs (14) ist, und ein drittes Befestigungsmittel (33) aufweist, das mit dem Befestigungsmittel (15) des Applikators (1) reversibel verbunden werden kann.

11. Applikator-Kit nach Anspruch 10, **gekennzeichnet durch**
einen Verschluss (31), mit dem die Öffnung des Mischbechers (3) verschließbar ist.

12. Applikator-Kit nach Anspruch 11, **dadurch gekennzeichnet, dass**
der Verschluss (31) ein Sperrschieber ist.

13. Applikator-Kit nach einem der Ansprüche 10 bis 12, **gekennzeichnet durch**
einen Mischlöffel (34) oder Mischspatel, der an einem Ende einen flächigen Stopfer besitzt, der senkrecht zur Achse des Mischlöffels oder Mischspatels steht und dessen Querschnittfläche kleiner oder gleich der Querschnittsfläche der Öffnung des Ausgaberohrs (32) Mischbechers ist.
